(19) Europäisches Patentamt
European Patent Office
Office européen des brevets

(11) **EP 4 296 651 A1**

(12) **EUROPÄISCHE PATENTANMELDUNG**

(43) Veröffentlichungstag:
**27.12.2023   Patentblatt 2023/52**

(21) Anmeldenummer: **23180659.7**

(22) Anmeldetag: **21.06.2023**

(51) Internationale Patentklassifikation (IPC):
**G01N 21/35** (2014.01)        **G06N 3/09** (2023.01)
**G01N 21/552** (2014.01)

(52) Gemeinsame Patentklassifikation (CPC):
**G06N 3/084; G01N 21/35; G01N 33/22; G06N 3/09;**
G01N 21/3504; G01N 21/552; G01N 2021/3595;
G01N 2201/1296; G06N 3/048

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(30) Priorität: **24.06.2022   DE 102022206362**

(71) Anmelder: **Karlsruher Institut für Technologie
76131 Karlsruhe (DE)**

(72) Erfinder:
• **Graf, David
76131 Karlsruhe (DE)**
• **Rauch, Reinhard
76139 Karlsruhe (DE)**
• **Asbahr, Wiebke
76131 Karlsruhe (DE)**

(74) Vertreter: **Müller-Boré & Partner
Patentanwälte PartG mbB
Friedenheimer Brücke 21
80639 München (DE)**

(54) **VERFAHREN UND VORRICHTUNG ZUR ERMITTLUNG DER RUSSTENDENZ EINER SUBSTANZ, VERWENDUNG DER VORRICHTUNG UND COMPUTERPROGRAMMPRODUKT**

(57)    Ein Aspekt der vorliegenden Erfindung betrifft ein Verfahren zur Ermittlung der Rußtendenz einer Substanz, insbesondere eines Kraftstoffs und/oder Treibstoffs, wobei das Verfahren die folgenden Schritte umfasst: Messen eines Spektrums der Substanz mittels eines Spektrometers, und Ermitteln, mittels einer Ermittlungseinheit, des Yield Sooting Index', YSI, der Substanz anhand des gemessenen Spektrums unter Verwendung einer angelernten Komponente maschinellen Lernens. Weitere Aspekte betreffen eine Vorrichtung zur Ermittlung der Rußtendenz einer Substanz, insbesondere eines Kraftstoffs, eine Verwendung der Vorrichtung zur Ermittlung der Rußtendenz einer Substanz, insbesondere eines Kraftstoffs, und ein Computerprogrammprodukt.

Figur 1

**Beschreibung**

Die vorliegende Erfindung betrifft ein Verfahren und eine Vorrichtung zur Ermittlung der Rußtendenz einer Substanz, eine Verwendung der Vorrichtung zur Ermittlung der Rußtendenz einer Substanz und ein Computerprogrammprodukt.

Der Yield Sooting Index (YSI) beschreibt die Rußtendenz (Emission von Partikeln) eines Reinstoffes oder eines Vielstoffgemisches bei der Verbrennung. Herkömmlicherweise muss dieser experimentell aufwendig gemessen werden. Die Rußbildungstendenz eines Moleküls oder Kraftstoffes kann über den YSI ausgedrückt werden, weshalb dieser ein wichtiger Parameter für die Entwicklung neuartiger Kraftstoffe mit geringen Partikelemissionen ist. Die Messung erfolgt mit einem sogenannten "Yale Coflow Burner". Im Experiment werden 1000 ppm der zu untersuchenden Probe in ein Methan/Stickstoff-Gemisch (55 % $CH_4$, 45 % $N_2$) bei atmosphärischem Druck dotiert. Die Probe kann ein Reinstoff, aber auch ein Vielstoffgemisch wie z. B. ein Kraftstoff sein. Der dotierte Gasstrom dient im Folgenden als Brennstoff für eine nicht vorgemischte laminare Flamme (Re ~ 60). Die Kraftstoffmischung tritt aus einer Röhre aus und reagiert mit der Umgebungsluft. Während des Verbrennungsvorgangs kommt es zur Bildung von Ruß. Die thermisch angeregten Partikel werden mittels "line-of-sight spectral radiance" (LSSR) als Strahlungssignal bei einer Wellenlänge von 660 nm erfasst. Das Resultat kann mittels einer linearen Beziehung (siehe nachfolgende Gleichung) in einen entsprechenden YSI umgerechnet werden. Als Referenzwerte in der Skala dienen hier Toluol (T) mit YSI = 170,9 und n-Heptan (H) mit YSI = 36,0.

$$YSI_{Probe} = (YSI_T - YSI_H) * \frac{LSSR_{Probe} - LSSR_H}{LSSR_T - LSSR_H} + YSI_H$$

Neben der LSSR-Methode wird zum Nachweis der Partikel auch auf die "laserinduced incandescence" (LII) zurückgegriffen. Der Versuchsaufbau bleibt bezüglich des Brenners unverändert. Die Dotierung fällt mit 400 ppm etwas geringer aus. Die Partikel in der Flamme werden bei der LII-Methode mit einem Laser thermisch angeregt. Wie bei der LSSR-Methode wird auch hier die freiwerdende Strahlung (400 - 455 nm) detektiert und mittels einer linearen Beziehung in einen YSI umgerechnet (siehe nachfolgende Gleichung).

$$YSI = C \cdot f_{v,max} + D$$

C und D sind apparatespezifische Parameter, welche mittels Referenzsubstanzen (analog zur Methode mit LSSR-Messtechnik) bestimmt werden. Als Referenzen dienen hier Benzol (YSI = 30) und 1 ,2-Dihydronaphtalin (YSI = 100).

Es kann auch auf andere Referenzen wie n-Hexan (YSI = 0) und Benzol (YSI = 100) zurückgegriffen werden. Somit ergeben sich verschiedene Datenbanken für den YSI, welche sich auf unterschiedliche Referenzen beziehen. Je nach Datenbank liegt der Fokus eher auf stark rußenden Substanzen wie Aromaten oder auf schwach rußenden wie Oxygenaten. Daher wurde eine öffentlich zugängliche, einheitliche Datenbank, die sogenannte "Unified Scale" entwickelt, indem die bestehenden Datenbanken einer neuen Regression unterzogen wurden. Die einheitliche Datenbank ist kalibriert auf die Werte $YSI_{n\text{-Hexan}}$ = 30 und $YSI_{Benzol}$ = 100. Für eine Großzahl von Reinstoffen ist der YSI abrufbar.

Neben der experimentellen Bestimmung besteht die Möglichkeit den YSI für Einzelsubstanzen mit ausreichender Genauigkeit mittels theoretischer Modelle vorherzusagen. Der YSI für Vielstoffgemische hingegen kann bisher nur begrenzt mit sogenannten Mischungsregeln rechnerisch abgeschätzt werden.

Eine experimentelle Bestimmung ist aufgrund des komplexen Versuchsaufbaus nicht ohne Weiteres in jedem Labor möglich. Die Beschaffung der Apparate ist kostenintensiv und verlangt einiges an Know-how bezüglich der eingesetzten Messtechnik.

Daher ist es Aufgabe der vorliegenden Erfindung ein Verfahren und eine Vorrichtung bereitzustellen, um den YSI effizienter ermitteln zu können.

Diese Aufgabe wird durch die unabhängigen Ansprüche gelöst. Besondere Ausführungsformen ergeben sich aus den abhängigen Ansprüchen.

Ein Aspekt der vorliegenden Erfindung betrifft ein Verfahren zur Ermittlung der Rußtendenz einer Substanz, insbesondere eines Kraftstoffs und/oder eines Treibstoffs, wobei das Verfahren die folgenden Schritte umfasst:

-  Messen eines Spektrums der Substanz mittels eines Spektrometers, und
-  Ermitteln, mittels einer Ermittlungseinheit, des Yield Sooting Index , YSI, der Substanz anhand des gemessenen Spektrums unter Verwendung einer angelernten Komponente maschinellen Lernens.

Vorteilhafterweise kann durch dieses Verfahren der YSI schneller, einfacher, kostengünstiger und genauer

ermittelt werden.

Weiter vorteilhafterweise können durch das erfindungsgemäße Verfahren Nichtlinearitäten in der Bestimmung des YSI von komplexeren Substanzen wie zum Beispiel Kraftstoffen berücksichtigt und/oder erkannt werden. Solche Substanzen enthalten nämlich in der Regel Hunderte von chemischen Komponenten, die sich gegenseitig beeinflussen und zu Nichtlinearitäten bei der Bestimmung des YSI führen können.

Des Weiteren müssen im Gegensatz zu herkömmlichen Bestimmungsmethoden Stoffmengenanteile der Einzelkomponenten nicht bestimmt werden bzw. bekannt sein. Die Konzentrationsinformation ist in dem gemessenen Spektrum enthalten und kann somit von der Komponente maschinellen Lernens in die Berechnungen mit einbezogen werden.

Die Anschaffung eines teuren Chromatographen und die zugehörige Methodenentwicklung zur quantitativen Aufklärung der Einzelsubstanzen entfallen damit.

Ein weiterer Vorteil des erfindungsgemäßen Verfahrens ist, dass im Gegensatz zu herkömmlichen Bestimmungsverfahren zum Beispiel mittels eines Yale-Brenners das erfindungsgemäße Verfahren einen einfacheren apparativen Aufbau zulässt.

Im Rahmen dieser Beschreibung schließt der Begriff "Messen" gemäß dem erfindungsgemäßen Verfahren das Bestimmen einer oder mehrerer physikalischer Größen wie zum Beispiel Intensität und/oder Wellenlänge und/oder Frequenz elektromagnetischer Strahlung ein.

Im Rahmen dieser Beschreibung bezieht sich der Begriff "Spektrum" auf die Gesamtheit aller von der Substanz absorbierten und/oder transmittierten elektromagnetischen Wellen bzw. Strahlung in einem bestimmten Wellenlängen- bzw. Frequenzbereich. Insbesondere bezieht sich der Begriff auf eine Intensitätsverteilung des von der Substanz absorbierten und/oder transmittierten Lichts in Abhängigkeit der Frequenz oder Wellenlänge des Lichts, wobei die Intensitätsverteilung diskrete, voneinander abgrenzbare Peaks aufweist, die elektromagnetischen Wellen einer bestimmten Frequenz bzw. Wellenlänge zugeordnet werden können, die von der Substanz absorbiert und/oder transmittiert werden. Das gemessene Spektrum kann sich aus Datenpaaren zusammensetzen, die die Wellenzahl oder Frequenz und Intensität der absorbierten und/oder transmittierten elektromagnetischen Strahlung beinhalten.

Im Rahmen dieser Beschreibung betrifft der Begriff "Spektrometer" eine optische Vorrichtung zum Messen eines wie oben beschriebenen Spektrums. Ein Spektrometer kann eine Lichtquelle, eine oder mehrere Linsen und ein lichtbrechendes bzw. -aufspaltendes Element wie zum Beispiel ein Prisma oder ein Beugungsgitter umfassen.

Im Rahmen dieser Beschreibung beziehen sich die Begriffe "Kraftstoff" und "Treibstoff" auf einen oder mehrere Stoffe, wie z. B. Benzin, Diesel oder Kerosin, durch dessen oder deren Verbrennung ein Motor und/oder eine Turbine angetrieben werden kann.

Im Rahmen dieser Beschreibung schließt der Begriff "Ermitteln" gemäß dem obengenannten Verfahren das Bestimmen und/oder Feststellen und/oder Vorhersagen ein.

Im Rahmen dieser Beschreibung bezieht sich der Begriff "Komponente maschinellen Lernens" auf das Gebiet "maschinelles Lernen" oder "Machine Learning".

Das maschinelle Lernen oder Machine Learning ist ein Teilgebiet im Bereich der künstlichen Intelligenz. Es beinhaltet die Anwendung und Wissenschaft von Algorithmen, die Muster in Daten erkennen und darin neue Erkenntnisse sammeln, die für den Menschen auf den ersten Blick nicht sichtbar sind. Machine Learning lässt sich in drei Methoden einteilen: überwachtes Lernen (engl. Supervised Learning), unüberwachtes Lernen (engl. Unsupervised Learning) und verstärktes Lernen (engl. Reinforcement Learning). Beim Supervised Learning soll das Modell mit Hilfe von Trainingsdaten lernen, Vorhersagen über unbekannte Daten zu treffen. Die Trainingsdaten beinhalten bereits den gewünschten Ausgabewert, welcher in der Programmiersprache mit "Labels" gekennzeichnet wird. Es gibt zwei generelle Typen von Aufgaben, die unter das Supervised Learning fallen: Klassifizierungs- und Regressionsprobleme. Bei Klassifizierungsproblemen geht es darum, anhand von Trainingsdaten die Klasse einer neuen Instanz vorherzusagen und somit eine Gruppierung durchzuführen. Im Gegensatz dazu werden bei Regressionsproblemen verschiedene Regressor-Variablen (abhängige und unabhängige Variablen) und eine Zielvariable (Ergebnis) vorgegeben und das Modell versucht, eine Beziehung zwischen diesen Variablen zu finden, um eine Vorhersage zu treffen.

Bei der Methode Unsupervised Learning gibt es im Gegensatz zum Supervised Learning keine Informationen über die Zielvariable. Hier geht es darum, durch die eingesetzten Verfahren aus Daten mit unbekannten Strukturen sinnvolle Informationen zu extrahieren. Für die Daten wird ein (Cluster-)Modell erstellt, welches eine Gruppierung durchführt, ohne zu wissen, wie die einzelnen Cluster heißen. Zu den Verfahren des Unsupervised Learnings zählt neben Datenanalyseverfahren (z. B. "Clustering") auch die Datenkomprimierung.

Vorzugsweise ist oder umfasst die Komponente maschinellen Lernens ein künstliches neuronales Netzwerk.

Insbesondere betrifft der Begriff "künstliches neuronales Netzwerk", der im Folgenden auch als "KNN" abgekürzt werden kann, die computergestützte Modellierung des menschlichen Gehirns.

Das menschliche Gehirn besteht aus miteinander verknüpften Nervenzellen, den Neuronen. Neuronen sind Berechnungseinheiten, die je nach Art des Neurons unterschiedliche Berechnungsschritte ausführen. In künstlichen neuronalen Netzwerken (im Folgenden auch als "künstliche neuronale Netze" bezeichnet) wird die Nervenzelle als sogenanntes Perzeptron modelliert. Das Perzeptron besteht aus einer Eingabeschicht (n-dimensionaler Vektor X), die ähnlich zu den Dendriten im Gehirn Reize von umgebenen Neuronen aufnehmen und an den Zellkörper weiterleiten. Der Zellkörper stellt im KNN die Berechnung der Übertragungsfunktion dar. Hier werden die Eingangssignale mit den jeweiligen Gewichtungen $W_i$ multipliziert und zusammengezählt. Häufig werden auch noch Grundgewichte, die sogenannten Bias, dazu addiert. Überschreitet im menschlichen Gehirn die berechnete Summe einen Schwellenwert, wird die Information über das Axon an ein anderes Neuron weitergegeben. Analog dazu wird in KNN die Summe an eine Aktivierungsfunktion weitergegeben, die den Ausgabewert berechnet. Es gibt eine Vielzahl von Aktivierungsfunktionen, die je nach Eingabe unterschiedliche Ergebnisse liefern können (z. B. einen Wert im Bereich von 0 bis 1).

In mehrschichtigen neuronalen Netzen findet besonders die sogenannte Rectifier-Funktion (Rectified Linear Unit - ReLU) Anwendung. Die Funktion ist definiert als:

$$f_{ReLU}(x) = max(0,x).$$

Schlussendlich erfolgt das Lernen des Perzeptrons durch die Anpassung der Gewichtungen. Dafür werden die Eingabewerte iterativ eingespeist und nach jedem Berechnungsschritt der Ausgabewert des Perzeptrons y' mit dem erwarteten Ausgabewert y verglichen.

Ein neuronales Netz umfasst eine Ansammlung vieler Neuronen mit gewichteten Verbindungen. Ein neuronales Netz bzw. Netzwerk weist mehrerer Schichten auf. Die erste Schicht wird als Input Layer bezeichnet. Hier werden die "Features" als Eingabevektor übergeben. Die verdeckten Schichten (engl. Hidden Layer) sind für die Weiterverarbeitung der Eingabeschicht zuständig. Existiert mehr als eine Hidden Layer kann

von tiefen neuronalen Netzen gesprochen werden (engl. Deep Neural Networks). Die Ausgabeschicht (engl. Output Layer) beinhaltet im Fall von Regressionsaufgaben die berechnete Vorhersage. Die einfachste Form eines KNN wird Multilayer Perzeptron genannt (MLP).

Im Lernprozess des neuronalen Netzes wird der Fehler, also die Abweichung zwischen

vorhergesagtem und tatsächlichen Ausgabewert durch eine Fehlerfunktion (engl. Loss Function) berechnet. Es gibt je nach Anwendungsbereich viele unterschiedliche Fehlerfunktionen. Für Regressionsprobleme werden typischerweise die Fehlerfunktionen "Mean Absolute Error" (MAE) oder "Mean Squared Error" (MSE) angewendet.

Nachdem der Fehler ermittelt wurde, werden die Gewichtungen mittels spezieller mathematischer Verfahren (wie z. B. dem Gradientenverfahren) angepasst. Die Anpassung beginnt bei der letzten Verbindung zwischen Output und vorhergehender Layer und schreitet nach und nach in Richtung Input Layer fort. Aufgrund dieser Rückkopplung wird von Backpropagation gesprochen. Findet das Training im Batchverfahren statt, erfolgt die Anpassung, nachdem eine bestimmte Anzahl an Trainingsbeispielen betrachtet wurde. Die Anzahl der betrachteten Trainingsbeispiele wird durch die "Batch size" definiert. Ein kompletter Durchlauf aller Trainingsdaten wird als "Epoche" bezeichnet. Die "Batch size" sowie die Anzahl an Epochen gehören im Deep Learning zu den essenziellen Hyperparametern, die beim Trainieren des Netzes eine wesentliche Rolle spielen.

Hyperparameter können zum Beispiel die Auswahl der richtigen Aktivierungsfunktion sowie Fehlerfunktion, Variablen, die bestimmen wie das neuronale Netz aufgebaut ist und die Batch size sowie Epoche sein. Im Lernprozess des künstlichen neuronalen Netzwerkes können zum Beispiel diese Hyperparameter optimiert werden.

Im Rahmen dieser Beschreibung kann das künstliche neuronale Netzwerk auch mit "KNN" abgekürzt werden oder sein.

Gemäß einer besonderen Ausführungsform umfasst das Verfahren ferner: Anlernen oder Trainieren der Komponente maschinellen Lernens, insbesondere mittels: gemessener Spektren einer Vielzahl von Referenzsubstanzen und/oder berechneter YSI-Werte der Vielzahl von Referenzsubstanzen.

Alternativ oder zusätzlich kann das Verfahren umfassen: Anlernen oder Trainieren der Komponente maschinellen Lernens, insbesondere mittels: berechneter Spektren der Vielzahl von Referenzsubstanzen und/oder gemessener YSI-Werte der Vielzahl von Referenzsubstanzen.

Vorzugsweise wird die Komponente maschinellen Lernens mithilfe von Trainingsdaten angelernt oder trainiert, die insbesondere obengenannten Parameter umfassen: die gemessenen Spektren einer Vielzahl von Referenzsubstanzen und/oder die, insbesondere theoretisch, berechneten und/oder gemessenen YSI-Werte der Vielzahl von Referenzsubstanzen.

Hierbei kann jede Referenzsubstanz der Vielzahl von Referenzsubstanzen bis zu acht, genau acht, oder mehr chemische Komponenten enthalten. Die chemischen Komponenten können Aromaten und/oder Paraffine und/oder Cycloalkane und/oder Oxygenate und/oder Olefine umfassen oder sein.

Als Referenzsubstanzen sind im Rahmen dieser Beschreibung insbesondere Substanzen zu verstehen, deren YSI-Werte bekannt und/oder mit verhältnismäßig geringem Rechenaufwand berechenbar und/oder verhältnismäßig einfach und schnell zu messen sind.

Zum Beispiel kann die Vielzahl von Referenzsubstanzen einen Reinstoff umfassen, dessen YSI experimentell und/oder durch theoretische Berechnungen bestimmt werden kann. Der YSI eines Reinstoffs kann auch von einer öffentlich zugänglichen Datenbank abgerufen werden oder der YSI eines Reinstoffs kann auch einer öffentlich zugänglichen Datenbank entnommen werden.

Eine solche Datenbank, wie die sogenannte "unified scale", kann zum Beispiel in MCENALLY, Charles S.; DAS, Dhrubajyoti D.; PFEFFERLE, Lisa D.: Yield Sooting Index Database Volume 2: Sooting Tendencies of a Wide Range of Fuel Compounds on a Unified Scale. 2007, Harvard Dataverse - https://doi.org/10.7910/DVN/7HGFT gefunden werden. Die sogenannte "low scale" Datenbank kann in MCENALLY, Charles S.; PFEFFERLE, Lisa D.: Sooting tendencies of oxygenated hydrocarbons in laboratory-scale flames. In: Environmental Science & Technology, Vol. 45, 2011, No. 6, S. 2498-2503. - ISSN 0013-936X; 1520-5851 (E). DOI: 10.1021/es103733q und DAS, Dhrubajyoti D.; MCENALLY, Charles S.; PFEFFERLE, Lisa D.: Sooting tendencies of unsaturated esters in nonpremixed flames. In: Combustion and Flame, Vol. 162, 2015, No. 4, S. 1489-1497. - ISSN 0010-2180 (P); 1556-2921 (E). DOI: 10.1016/j.combustflame.2014.11.012 gefunden werden. Die sogenannte "high scale" Datenbank kann in MCENALLY, Charles S.; PFEFFERLE, Lisa D.: Improved sooting tendency measurements for aromatic hydrocarbons and their implications for naphthalene formation pathways. In: Combustion and Flame, Vol. 148, 2007, No. 4, S. 210-222. - ISSN 0010-2180 (P); 1556-2921 (E). DOI: 10.1016/j.combustflame.2006.11.003 und MCENALLY, Charles S.; PFEFFERLE, Lisa D.: Sooting tendencies of nonvolatile aromatic hydrocarbons. In: Proceedings of the Combustion Institute, Vol. 32, 2009, No. 1, S. 673-679. - ISSN 1540-7489 (P); 1873-2704 (E). DOI: 10.1016/j.proci.2008.06.197 gefunden werden.

Der YSI eines Reinstoffs kann zum Beispiel mittels eines theoretischen Modells berechnet bzw. ermittelt werden, das auf dem Zerlegen des Reinstoffs in einzelne Molekülgruppen basiert. Ein Molekül kann in sogenannte "Deskriptoren" bzw. Molekülfragmente zerlegt werden, die jeweils einen YSI zum gesamten Molekül beitragen. Diese Werte können zuvor zum Beispiel mittels Regression bestimmt worden sein.

Insbesondere können Moleküle nach der Methode von Benson & Buss in Fragmente zerlegt werden. Der YSI eines gesamten Moleküls kann mit einer Bayes'schen multivariaten linearen Regression ermittelt werden (siehe zum Beispiel DAS, Dhrubajyoti D. [u.a.]: Measuring and predicting sooting tendencies of oxygenates, alkanes, alkenes, cycloalkanes, and aromatics on a unified scale. In: Combustion and Flame, Vol. 190, 2018, S. 349-364. ISSN 0010-2180 (P); 1556-2921 (E). DOI: 10.1016/j.combustflame.2017.12.005.).

Ferner kann der YSI eines Reinstoffs mit dem online verfügbaren "YSI Estimator" bestimmt werden. Der "YSI Estimator" basiert auf dem Zerlegen von Molekülen in einzelne Bruchstücke. Das Modell des "YSI Estimators" beruht auf einer Gruppenbeitragsmethode. Mit diesem Softwarewerkzeug kann durch Zeichnen der einzelnen Komponenten des Reinstoffes oder durch Eingeben einer SMILES-Zeichenkette ("Simplified Molecular Input Line Entry Specification") der YSI des Reinstoffs erhalten werden. Falls für den Reinstoff auch ein experimentell gemessener YSI existiert, kann dieser durch das Softwarewerkzeug auch aus- bzw. angegeben werden.

Alternativ oder zusätzlich kann die Vielzahl von Referenzsubstanzen einen Reinstoff umfassen, dessen Spektrum experimentell und/oder durch theoretische Berechnungen bestimmt werden kann. Das Spektrum eines Reinstoffes kann über klassische und/oder quantenmechanische Modelle zur Schwingung und Strahlungsabsorption von Molekülen berechnet werden.

Ein Spektrum, insbesondere ein Infrarotspektrum, eines Reinstoffs kann zum Beispiel quantenmechanisch im Rahmen der Dichtefunktionaltheorie oder semiempirisch mittels eines Additionsschemas berechnet bzw. bestimmt werden.

Alternativ oder zusätzlich kann die Vielzahl von Referenzsubstanz eine Mischung aus zum Beispiel 2, 3, 4, 5, 6, 7, 8 oder mehr Reinstoffen umfassen, deren YSI-Werte und/oder Spektren experimentell und/oder durch theoretische Berechnungen bestimmt werden können.

Das Spektrum eines Stoffgemisches kann über klassische und/oder quantenmechanische Modelle zur Schwingung und Strahlungsabsorption von Molekülen berechnet werden. Insbesondere ist es möglich, das Spektrum eines Stoffgemischs basierend auf den Einzelspektren der das Stoffgemisch bildenden Reinstoffe zu bestimmen. Beispielsweise kann das Spektrum des Stoffgemischs das Summenspektrum der Spektren der Reinstoffe sein. Insbesondere kann das Spektrum des Stoffgemischs das gewichtete Summenspektrum der Spektren der Reinstoffe, insbesondere einer gewichteten Linearkombination der Spektren der Reinstoffe sein.

Das Gesamtspektrum $s$ eines Stoffgemischs kann nach dem Lambert-Beer'schen Gesetz aus den Einzelspektren $K_i$ wie folgt berechnet werden:

$$s = \sum_i y_i \cdot K_i$$

Der Parameter $y_i$, der einem Konzentrationsmaß entsprechen kann, kann so gewählt werden (z. B. durch Iteration), dass s das gemessene Spektrum bestmöglich beschreiben kann. Bei bekannten Konzentrationen in einer Probe kann $y_i$ auch direkt berechnet werden.

Der YSI einer Mischung aus Reinstoffen kann bei bekannten YSI-Werten $YSI_i$ der einzelnen Reinstoffe (i = 2, 3, 4, 5, 6, 7, 8, ...) theoretisch berechnet werden, wie folgt:

Basierend auf den Werten der Einzelkomponenten bzw. der einzelnen Reinstoffe $YSI_i$ kann der YSI einer Mischung $YSI_{mix}$ mittels der Mischungsregel in nachfolgender Gleichung abgeschätzt oder näherungsweise berechnet werden.

$$YSI_{mix} = \sum_i x_i \cdot YSI_i \qquad\qquad (3)$$

Diese Mischungsregel wurde für Mischungen aus bis zu acht Reinstoffen bestätigt. Für die Anwendbarkeit der Mischungsregel müssen die Stoffmengenanteile $x_i$ der einzelnen Komponenten bzw. Reinstoffe bekannt sein.

Die Trainingsdaten zum Anlernen der Komponente maschinellen Lernens können demnach gemessene und/oder berechnete Spektren von Reinstoffen und/oder Stoffmischungen enthalten. Zudem können die Trainingsdaten die den Spektren bzw. Referenzsubstanzen entsprechenden YSI-Werte enthalten, die gemessen und/oder berechnet worden sein können.

Beispielsweise kann ein Stoffgemisch mit bis zu 8 Reinstoffen hergestellt, insbesondere zusammengemischt werden. Das Spektrum dieses Stoffgemischs kann experimentell bestimmt werden, d.h. gemessen werden. Der YSI dieses Stoffgemischs kann gemäß obigem Modell nach Gleichung (3) bestimmt werden, sofern der jeweilige YSI-Wert der Reinstoffe bekannt ist. Diese Daten können zum Training (inklusive Validieren) der Komponente maschinellen Lernens verwendet werden. Vorzugsweise werden eine Vielzahl solcher Daten, d.h. Spektren von Stoffgemischen und zugeordnetem YSI zum Training (inklusive Validieren) erzeugt.

Alternativ oder zusätzlich kann das Spektrum eines Stoffgemischs mit bis zu 8 Reinstoffen mathematisch erstellt werden. Sofern die Spektren der Reinstoffe bekannt sind, kann das Spektrum des Stoffgemischs das (gewichtete) Summenspektrum der Spektren der Reinstoffe, insbesondere eine (gewichtete) Linearkombination der Spektren der Reinstoffe sein. Der YSI dieses Stoffgemischs kann gemäß obigem Model nach Gleichung (3) bestimmt werden, sofern der jeweilige YSI-Wert der Reinstoffe bekannt ist. Diese Daten können zum Training (inklusive Validieren) der Komponente maschinellen Lernens verwendet werden. Vorzugsweise werden eine Vielzahl solcher Daten, d.h. Spektren von Stoffgemischen und zugeordnetem YSI zum Training (inklusive Validieren) erzeugt.

Alternativ zu den vorangehenden Ausführungen kann der YSI des Stoffgemischs auch experimentell bestimmt, d.h. gemessen werden, z.B. mit eine Yale Coflow Burner, wie oben ausgeführt. Diese Daten können zum Training (inklusive Validieren) der Komponente maschinellen Lernens verwendet werden. Vorzugsweise werden eine Vielzahl solcher Daten, d.h. Spektren von Stoffgemischen und zugeordnetem YSI zum Training (inklusive Validieren) erzeugt.

Es ist auch möglich, dass das Stoffgemisch aus mehr als 8 Reinstoffen besteht. Das Spektrum des Stoffgemischs kann experimentell bestimmt werden, d.h. gemessen werden oder mathematisch bestimmt werden, wie bereits oben ausgeführt wurde. DerYSI kann experimentell bestimmt, d.h. gemessen werden oder, sofern verfügbar, mathematisch über ein geeignetes Modell bestimmt werden. Diese Daten können zum Training (inklusive Validieren) der Komponente maschinellen Lernens verwendet werden. Vorzugsweise werden eine Vielzahl solcher Daten, d.h. Spektren von Stoffgemischen und zugeordnetem YSI zum Training (inklusive Validieren) erzeugt.

Die Trainingsdaten, insbesondere die die Spektren der Referenzsubstanzen umfassenden Daten bzw. die Spektraldaten der Referenzsubstanzen, können komprimiert oder reduziert werden. Diese Komprimierung oder Reduzierung kann durch eine Hauptkomponentenanalyse erfolgen. Hierdurch kann der Anlern- bzw. Trainingsvorgang der Komponente maschinellen Lernens beschleunigt werden, weil die Rechenzeit reduziert werden kann.

Der Trainingsablauf kann zum Beispiel wie folgt sein: Die Features der Trainingsdaten werden in die Struktur zum Beispiel eines neuronalen Netzes iterativ eingespeist. Die Initialisierung der Gewichtungen und Bias erfolgt zunächst zufällig. Durch Berechnung der Aktivierungsfunktionen durchlaufen die Daten nach dem Feed-Forward-Prinzip das neuronale Netz von der Input- bis zur Output Layer. Die Output Layer gibt die Vorhersage des Indexes aus. Die Abweichung zwischen vorhergesagtem und experimentellen YSI wird mit der Loss-Funktion berechnet. Das Ziel des Trainings ist die Loss-Funktion so weit wie möglich zu reduzieren. Um dies zu erreichen, werden die Gewichtungen durch Backpropagation angepasst. Die Anpassung der Gewichtungen erfolgt nach der Einspeisung jedes Datensatzes oder nach einer bestimmten Batch size. Dieser Vorgang wird so lange durchgeführt, bis die Anzahl an vorgegebenen Epochen erreicht ist oder erreicht werden.

Das Anlernen oder Trainieren kann einmal oder mehrmals wiederholt werden.

Alternativ oder zusätzlich kann das Verfahren ferner umfassen: Weiteranlernen oder Weitertrainieren der Komponente maschinellen Lernens mittels des gemessenen Spektrums der Substanz und/oder des ermittelten YSI der Substanz. Mit anderen Worten kann die Datenmenge, mit der die Komponente maschinellen Lernens angelernt oder trainiert wird, erweitert werden, um beispielsweise die Präzision der Komponente maschinellen Lernens zu verbessern und/oder die Anwendbarkeit der Komponente maschinellen Lernens auf eine größere Menge von Stoffgruppen zu erweitern. Die Datenmenge kann insbesondere nachträglich erweitert werden.

Das Weiteranlernen oder Weitertrainieren kann einmal oder mehrmals wiederholt werden.

Das Weiteranlernen oder Weitertrainieren kann einschließen, dass die Komponente maschinellen Lernens iterativ angelernt oder trainiert wird. Ferner kann das Weiteranlernen oder Weitertrainieren einschließen, dass die Komponente maschinellen Lernens erneut mit dem ermittelten oder vorhergesagten YSI-Wert der Substanz weiter angelernt oder trainiert wird, um die Vorhersage des YSI-Wertes durch die Komponente maschinellen Lernens zu verbessern.

Vorteilhafterweise kann durch das oben beschriebene An- und/oder Weiteranlernen bzw. Trainieren und/oder Weitertrainieren des künstlichen neuronales Netzwerkes zum einen die Ermittlung des YSI-Wertes einer Substanz verbessert werden. Zum anderen können die YSI-Werte für komplexere Substanzen, insbesondere Substanzen, die mehr als 100 Komponenten aufweisen können, einfach und schnell ermittelt werden.

Gemäß einer besonderen Ausführungsform ist das Spektrum der Substanz ein Absorptionsspektrum, insbesondere ein Infrarotabsorptionsspektrum, und/oder ein Transmissionsspektrum, insbesondere ein Infrarottransmissionsspektrum.

Als Absorptionsspektrum wird hier ein Spektrum bezeichnet, dass der von der Substanz nicht durchgelassenen bzw. absorbierten elektromagnetischen Wellen bzw. Strahlung im Verhältnis zur auf die Substanz einfallenden Strahlung entspricht, wobei in der transmittierten Strahlungsverteilung die absorbierten Wellenlängen- bzw. Frequenzbereiche fehlen.

Als Transmissionsspektrum wird hier ein Spektrum bezeichnet, dass der von der Substanz durchgelassenen bzw. nicht absorbierten oder transmittierten elektromagnetischen Wellen bzw. Strahlung im Verhältnis zur auf die Substanz einfallenden Strahlung entspricht, wobei in der transmittierten Strahlungsverteilung die absorbierten Wellenlängen- bzw. Frequenzbereiche fehlen.

Die Infrarotspektroskopie basiert auf Wechselwirkungen von elektromagnetischer Strahlung mit Materie. Durch die Absorption von Strahlung im infraroten Bereich werden Moleküle zu Schwingungen und Rotation angeregt. Die Frequenz der absorbierten Strahlung ist abhängig von der Art der Schwingung, welche durch die Struktur der untersuchten Verbindung beeinflusst wird. Infrarotspektren können aufgenommen werden, indem die Probe mit infrarotem Licht bestrahlt wird und das Verhältnis der Intensitäten des austretenden und einfallenden Strahls untersucht wird. Die Durchlässigkeit (Transmittance - T) wird über die Wellenlänge bzw. über den reziproken Wert, der Wellenzahl, aufgetragen und gilt als Maß für die Energieaufnahme der Probe.

Die Schwingungen und die dadurch entstehenden Absorptionsbanden in definierten Bereichen im Infrarotspektrum sind charakteristisch für viele funktionelle Gruppen organischer Moleküle. Ein komplexes Molekül kann eine Vielzahl von Schwingungen aufweisen. Die sogenannten Normalschwingungen, die durch die Freiheitsgrade der drei unabhängigen Raumrichtungen entstehen, lassen sich in Valenz- und Deformationsschwingungen einteilen.

Valenzschwingungen sind Bewegungen entlang der Bindungsachse, wobei sich die Bindungslängen verändern. Als Deformationsschwingung werden Schwingungen bezeichnet, bei denen sich die Bindungswinkel ändern, die Bindungsabstände jedoch nahezu gleichbleiben. Ein IR-Spektrum kann in zwei größere Bereiche aufgeteilt werden. Im Wellenzahlbereich oberhalb 1500 cm$^{-1}$ befinden sich Valenzschwingungen. Diese Absorptionsbanden können den funktionellen Gruppen zugeordnet werden. Im Bereich darunter treten Deformationsschwingungen sowie Kombinationsschwingungen auf. Kombinationsschwingungen resultieren aus der Absorption von zwei oder mehreren Normalschwingungen und sind charakteristisch für das Molekül als Ganzes. Dieser Bereich wird deshalb auch als "fingerprint"-Region bezeichnet

Gemäß einer besonderen Ausführungsform umfasst das gemessene Spektrum der Substanz bis zu ungefähr 3500 Stützstellen. Das gemessene Spektrum kann genau 3500 Stützstellen aufweisen. Das gemessene Spektrum kann mindestens 100 Stützstellen und höchstens 3500 Stützstellen aufweisen. Das gemessene Spektrum kann zwischen 100 und 500, 500 und 1000, 1000 und 1500, 1500 und 2000, 2000 und 2500, 2500 und 3000, und 3000 und 3500 Stützstellen aufweisen. Das gemessene Spektrum kann auch mehr als 3500 Stützstellen aufweisen.

Die Stützstellen können den Wellenzahlen der von der Substanz absorbierten und/oder transmittierten elektromagnetischen Strahlung entsprechen. Die Wellenzahlen sind vorzugsweise im Bereich von ungefähr 4000 bis ungefähr 400 cm$^{-1}$. Die Wellenzahl kann sich aus der entsprechenden Wellenlänge der elektromagnetischen Strahlung wie folgt ergeben: $k\ [cm^{-1}] = 10^4/(\lambda\ [\mu m])$.

Die Anzahl der Stützstellen bzw. Datenpunkte des gemessenen Spektrums der Substanz entspricht vorzugsweise der Anzahl an Stützstellen bzw. Datenpunkten, die die gemessenen und/oder berechneten Spektren der Referenzsubstanzen der Vielzahl von Referenzsubstanzen umfassen, die zum Anlernen der Komponente maschinellen Lernens verwendet werden können.

Es können zum Beispiel 1762 Stützstellen zwischen der Wellenzahl 500 und 4000 sein. Das gemessene Spektrum kann interpoliert werden, so dass jedes Spektrum die gleiche Anzahl von Stützstellen aufweist. Das interpolierte Spektrum kann zum Beispiel 3500 Stützstellen aufweisen.

Gemäß einer besonderen Ausführungsform ist das Spektrometer ein Infrarotspektrometer, insbesondere ein Fourier-Transform-Infrarotspektrometer (im Folgenden bezieht sich die Abkürzung "FT" auf "Fourier-Transform").

Im Gegensatz zur normalen Infrarotspektroskopie (im Folgenden bezieht sich die Abkürzung "IR" auf "infrarot") nutzt die FT-IR-Spektroskopie infrarotes Licht über einen großen Wellenlängenbereich. Das gemessene Interferogramm lässt sich anschließend durch einfache Fourier-Transformation wieder in ein Spektrum umwandeln. Diese Vorgehensweise bringt mehrere Vorteile mit sich. Zum einen ist das Aufnehmen der Spektren deutlich schneller (Sekunden statt Minuten) und zum anderen zeigen die Spektren eine höhere Wellenlängengenauigkeit. Zudem wird bei der Messung die ganze Intensität der Lichtquelle genutzt, wodurch ein verbessertes Signal-Rausch-Verhältnis entsteht.

Weiter insbesondere kann das Spektrometer ein Abgeschwächte-Totalreflexion-Fourier-Transform-Infrarotspektrometer sein.

Die abgeschwächte Totalreflexion (im Folgenden als "ATR" abgekürzt) ist eine Messtechnik der IR-Spektroskopie. Der erzeugte Lichtstrahl läuft durch einen Kristall mit einer hohen Brechkraft. Aufgrund der Wellennatur des Lichts dringt dieser ein wenig in die optisch "dünnere" Messprobe ein. Durch die Absorption im optisch "dünneren" Medium wird die Intensität des reflektierenden Lichts, abhängig von der Wellenlänge, teilweise geschwächt. Dadurch lassen sich Rückschlüsse auf das Medium ziehen.

Der Vorteil dieser Messtechnik ist, dass sich Proben in unterschiedlichsten Aggregatszuständen ohne Vorbehandlung vermessen lassen.

Ein weiterer Vorteil der Verwendung eines ATR-FT-IR-Spektrometers ist, dass der YSI einer Substanz zerstörungsfrei, d. h. ohne größere Mengen der Substanz zu verbrauchen, ermittelt werden kann. Mit anderen Worten wird bei der spektrometrischen Analyse durch ein ATR-FT-IR-Spektrometer nur ein Tropfen der zu untersuchenden Substanz benötigt.

Gemäß einer besonderen Ausführungsform umfasst das Verfahren ferner: Übertragen des gemessenen Spektrums der Substanz von dem Spektrometer an die Ermittlungseinheit.

Alternativ oder zusätzlich umfasst das Verfahren ferner: Empfangen des gemessenen Spektrums der Substanz durch die Ermittlungseinheit.

Alternativ oder zusätzlich umfasst das Verfahren ferner: Einlesen des gemessenen Spektrums der Substanz in die Ermittlungseinheit.

Das gemessene Spektrum kann in eine computerlesbare Datei umgewandelt werden oder sein und/oder in einer computerlesbaren Datei abgelegt und/oder gespeichert werden oder sein. Die das gemessene Spektrum enthaltende Datei kann über eine Verbindung zwischen dem Spektrometer und der Ermittlungseinheit, wie einem Netzwerk, zum Beispiel ein LAN oder WLAN, übertragen und/oder empfangen werden. Die Übertragung kann mittels JSON stattfinden. Die das gemessene Spektrum enthaltende Datei kann auf einem Datenträger gespeichert werden oder sein. Die Datei kann über die Verbindung oder von dem Datenträger in die Ermittlungseinheit eingelesen werden.

Gemäß einer besonderen Ausführungsform umfasst die Ermittlungseinheit oder ist die Ermittlungseinheit eine Computereinheit, die zumindest einen Prozessor und zumindest eine Speichereinheit aufweist, wobei die zumindest eine Speichereinheit, die Komponente maschinellen Lernens, insbesondere ein künstliches neuronales Netzwerk, umfasst.

Die Ermittlungseinheit kann dazu ausgelegt sein, den YSI einer Substanz mithilfe der Komponente maschinellen Lernens, insbesondere des künstlichen neuronalen Netzwerkes, auszuwerten.

Der zumindest eine Prozessor kann einen oder mehrere CPUs und/oder GPUs umfassen. Die zumindest eine Speichereinheit kann eine oder mehrere verbundene Festplatten und/oder einen Cloud-Speicher umfassen.

Alternativ oder zusätzlich kann das Spektrometer zumindest einen Prozessor und zumindest eine Speichereinheit umfassen.

Gemäß einer besonderen Ausführungsform umfasst die Ermittlungseinheit einen Desktop-PC und/oder ein Smartphone und/oder einen Tablet-PC. Alternativ kann die Ermittlungseinheit ein Desktop-PC und/oder ein Smartphone und/oder ein Tablet-PC sein.

In dieser Ausführungsform kann das Verfahren in bestehende Laborprozesse einfach integriert oder implementiert werden.

Gemäß einer besonderen Ausführungsform umfasst das Verfahren ferner: Durchführen einer Datenreduktion des gemessenen Spektrums der Substanz, insbesondere einer Hauptkomponentenanalyse des gemessenen Spektrums der Substanz.

Mittels einer Datenreduktion können zum Beispiel die Anzahl der Stützstellen von ungefähr 3500 auf ungefähr 100 reduziert werden, wodurch sich die Größe der das gemessene Spektrum enthaltenden Datei effektiv verkleinern lässt. Dadurch kann das zuvor beschriebene Verfahren schneller und mit erhöhter Genauigkeit ablaufen. Mit anderen Worten kann der YSI einer Substanz schneller und genauer ermittelt werden.

Gemäß einer besonderen Ausführungsform umfasst das Verfahren ferner: Bereitstellen der Substanz.

Das Bereitstellen der Substanz kann einschließen, dass die Substanz in das Spektrometer eingebracht und/oder in dem Spektrometer angeordnet wird.

Gemäß einer besonderen Ausführungsform umfasst das Verfahren ferner: Ausgeben des ermittelten YSI der Substanz. Der YSI kann zum Beispiel über einen Bildschirm an der Ermittlungseinheit ausgegeben werden.

Gemäß einer besonderen Ausführungsform umfasst oder ist die Substanz ein Reinstoff oder ein Stoffgemisch, wobei die Substanz insbesondere einen Kraftstoff und/oder einen Treibstoff umfasst oder ein Kraftstoff und/oder ein Treibstoff ist. Die Substanz kann zum Beispiel Benzin, Diesel, Kerosin oder Ähnliches umfassen oder sein.

Das oben beschriebene Verfahren kann von einem Computer oder einem Computersystem ausgeführt werden. Das Computersystem kann das Spektrometer und die Ermittlungseinheit umfassen.

Das Verfahren gemäß dem obengenannten Aspekt der Erfindung kann in folgender Reihenfolge ablaufen oder ausgeführt werden: In einem ersten Schritt kann das Spektrum der Substanz gemessen werden. In einem nächsten, insbesondere zweiten oder letzten, Schritt kann der YSI der Substanz ermittelt werden.

In einer Ausführungsform des Verfahrens kann das Verfahren die folgende Schrittreihenfolge umfassen: Bereitstellen der Substanz, Messen des Spektrums der Substanz mittels des Spektrometers, Übertragen des gemessenen Spektrums der Substanz von dem Spektrometer an die Ermittlungseinheit und/oder Einlesen des gemessenen Spektrums der Substanz in die Ermittlungseinheit, Ermitteln, mittels der Ermittlungseinheit, des YSI der Substanz anhand des gemessenen Spektrums unter Verwendung der angelernten Komponente maschinellen Lernens, und optional Darstellen des ermittelten YSI der Substanz über einen Bildschirm, der zum Beispiel an der Ermittlungseinheit angeordnet sein kann.

Der Schritt des Ermittelns des YSI der Substanz in dem oben beschriebenen Verfahren kann ferner einschließen: Anlernen der Komponente maschinellen Lernens, die ein künstliches neuronales Netzwerk umfasst oder ist, und Auswerten des gemessenen Spektrums der Substanz mittels des künstlichen neuronalen Netzwerks.

Im Folgenden wird beispielhaft beschrieben, wie die angelernte Komponente des maschinellen Lernens, insbesondere das angelernte künstliche neuronale Netzwerk, implementiert und initialisiert werden kann:
Zu Beginn können alle benötigten Bibliotheken importiert werden. Dazu können die Bibliotheken Pandas, TensorFlow, Keras, NumPy, Scikit-learn, Matplotlib und viele weitere gehören. Die Implementierung kann auf der Programmiersprache Python basieren.

Als nächstes können die Modelldaten für das künstliche neuronale Netzwerk (im Folgenden in seiner Gesamtheit auch als "Modell" bezeichnet) eingelesen werden. Die Datenpunkte jedes Spektrums können in einzelnen JCAMP-DX-Dateien abgespeichert sein. Die Namen der Dateien und die entsprechenden YSI können in einer separaten Excel-Datei zugeordnet sein. Diese Excel-Datei kann in einen "Pandas DataFrame" eingelesen werden. Unter "y" können die Zielvariablen, also die YSI abgespeichert werden. Eine Liste aller Spektren-Dateinamen kann erstellt werden und die Anzahl aller Daten, die Trainings- und Testdaten umfassen, kann bestimmt werden. In einer Funktion "x_Einlesen" kann die Methode "JCAMP_reader" der Bibliothek Jcamp aufgerufen werden. Diese Methode kann es ermöglichen, die x-Werte (Wellenzahlen) bzw. y-Werte (Absorptionswerte) eines Spektrums, zum Beispiel abgespeichert in einer JCAMP-DX-Datei, einzulesen. Die x-Werte können in einer Liste abgespeichert werden und die Werte getauscht werden, so dass die Liste mit der niedrigsten Wellenzahl beginnen kann. Die Werte können mit ihren jeweiligen Nachkommastellen eingelesen werden. Anschließend kann zwischen den Punkten interpoliert werden, um die Absorption für eine vorher festgelegte Anzahl von ganzen Wellenzahlen zu erhalten, zum Beispiel eine genaue Absorption bei einer Wellenzahl von 2900, wenn die tatsächlichen Messwerte bei 2989,7 und 2901,8 liegen. Mit einer Funktion "y_Einlesen" können die y-Werte eingelesen werden.

Die Interpolation kann zum Beispiel mit der Methode "np.interp" von NumPy durchgeführt werden. Es kann interpoliert werden, da für eine anschließende Hauptkomponentenanalyse von jedem Spektrum die Absorptionswerte von den gleichen Wellenzahlen bzw. Stützstellen benötigt werden. Anschließend können alle negativen Absorptionswerte, entstanden durch leichte Schwankungen um die Null-Linie, mit der "np.maximum" Methode auf null gesetzt werden. Die Absorptionswerte aller eingelesenen Stoffe können in einem Pandas DataFrame gespeichert werden. Vorliegend können zum Beispiel 237 Proben eingelesen worden sein. Die Datenpunkte können auch als Features bezeichnet werden, sofern sie direkt ohne weitere Verarbeitung in das KNN eingespeist werden können.

Als Nächstes kann eine Hauptkomponentenanalyse (engl. "Principal Component Analysis"; kurz: "PCA") durchgeführt werden. Diese Methode kann einfach von der Bibliothek Scikit-learn implementieren werden. Hierbei kann die Anzahl an Hauptkomponenten bzw. PC (kurz für "Principal Component(s)") festgelegt werden. Die PC können dann für jeden eingelesenen Stoff in einem DataFrame gespeichert werden.

Die Features und Zielvariablen können nun in Trainings- und Testdaten gesplittet werden. Mit der Methode "train_test_split" von Scikit-learn können die Daten in 20 % Testdaten und 80 % Trainingsdaten gesplittet werden. Mit der Anweisung "shuffle=True" können die Daten durchmischt und zufällig eingeteilt werden. Durch das Angeben einer Zahl bei "random_state" kann festgelegt werden, dass bei gleicher Zahl immer die gleichen Daten in Test- und Trainingsdaten aufgeteilt werden. So kann eine Wiederholbarkeit der Ergebnisse erzielt werden. In "X_train" bzw. "X_test" können die Features gespeichert werden, in "Y_train" bzw. "Y_test" die Zielvariablen (YSI).

Als Nächstes kann die Struktur des neuronalen Netzes festgelegt werden. Die Initialisierung der Gewichtungen kann mit dem Default-Initialisierer "GlorotUniform" erfolgen. Mit dem Befehl "seed" kann analog zur "train_test_split"-Methode festgelegt werden, dass bei jedem Aufruf die gleichen Gewichtungen initialisiert werden. Es können zum Beispiel zwei "Dense-Layer" mit dazugehöriger Anzahl an Neuronen, Aktivierungsfunktion und Initialisierer definiert werden. Mit Keras kann automatisch eine Input Layer mit der Anzahl an Neuronen identisch zur Anzahl an Features erzeugt werden, sofern diese Layer nicht manuell definiert werden. In der "compile"-Methode von Keras kann das Modell für den YSI nun durch Angabe des Optimierers, der Loss-Funktion und der Metriken final konfiguriert werden. Die Metriken können als mittlerer absoluter Fehler in Prozent und mittlerer absoluter Fehler definiert werden.

Nach der Vorverarbeitung der Daten und der Definition des neuronalen Netzes kann das Modell trainiert werden. Das eigentliche Training des Modells kann durch die "fit"-Methode von Keras gestartet werden. Dafür können die Trainings-Features ("x_train_arr"), Trainings-Labels ("y_train_arr"), Validierungsdaten, Epochen und die Batch size angegeben werden. Die Fehler während des Trainingsvorgangs können in einem Dictionary "history1" gespeichert werden und können durch die "history"-Methode von Keras ausgegeben werden. Für die Optimierung des Modells und dessen Hyperparameter kann eine k-fache Kreuzvalidierung eingeführt werden.

Als Nächstes können die Methoden "KFold" und "split" von Scikit-learn aufgerufen werden. Diese Methode kann mit Angabe der Splits die Trainingsdaten ("X_train") zufällig zum Beispiel in zehn gleich große Datensets aufteilen. In jedem Durchlauf ("for-Schleife") kann die "fit"-Methode nun auf andere Trainings- und Validierungsdaten zugreifen, so dass zum Beispiel zehn unabhängige Modelle trainiert werden können. Die Fehler, gespeichert in "history1", aller Modelle können gemittelt werden und mit der Bibliothek Matplotlib dargestellt werden. Um das Modell mit der besten Vorhersagefähigkeit zu ermitteln, können zunächst die Hyperparameter verändert werden und die Einstellungen mit der niedrigsten Loss-Funktion der Validierungsdaten gesucht werden.

Mit zum Beispiel einer Gitterstudie oder einer ähnlichen oder anderen Optimierungsmethode kann eine Feinabstimmung der Hyperparameter durchgeführt werden. Ein Vorteil besteht darin, dass verschiedene Hyperparameter gleichzeitig verändert werden können. Dies kann sinnvoll sein, da einige Hyperparameter voneinander abhängig sein können. Da das Modell mit Keras konstruiert wurde und die Gitterstudie mit Scikit-learn durchgeführt werden kann, kann zunächst eine Funktion erstellt werden, die das Keras-Modell definiert und ausgibt. Ein Dictionary "param_grid" kann das Gitter für die "Grid Search" darstellen. In dieser Gitterstudie können nun Lernrate und Epochen variiert werden und für alle möglichen Kombinationen der mittlere absolute Fehler in Prozent ausgegeben werden. Auch die Struktur des neuronalen Netzes, insbesondere die Anzahl an Lagen bzw. "Layer" sowie die Anzahl an Neuronen pro Lage bzw. "Layer", kann variiert bzw. optimiert werden. Im Gegensatz zur "Grid Search" kann bei der "Randomized Search" eine Parameter-Verteilung angegeben ("param_dist") werden. Hier können die Anzahl an Kombinationen ("n_iter"), zum Beispiel 100, vorgegeben werden, die zufällig ausgewählt werden.

In einem nächsten, insbesondere letzten Schritt der Implementierung kann die finale Einschätzung der Vorhersage-Fähigkeit des Modells mithilfe von Testdaten, die einem Teil aller zum Training des KNN verwendbaren Daten entsprechen können, stattfinden. Dafür kann das Modell erneut mit den optimalen Hyperparametern mit der "fit"-Methode trainiert werden. Der Unterschied zum vorherigen Aufruf kann darin bestehen, dass dieses Mal alle Trainingsdaten für das Training genutzt werden. Mit der "evaluate"-Methode von Keras kann das Modell hinsichtlich der Testdaten evaluiert werden. Zudem können mit der "predict"-Methode Modell-Vorhersagen der Testdaten ausgegeben werden.

Ein weiterer Aspekt der vorliegenden Erfindung betrifft eine Vorrichtung zur Ermittlung der Rußtendenz einer Substanz, insbesondere eines Kraftstoffs und/oder eines Treibstoffs, umfassend:

- ein Spektrometer zur Messung eines Spektrums der Substanz, und
- eine Ermittlungseinheit, die dazu ausgelegt ist, den Yield Sooting Index, YSI, der Substanz anhand des gemessenen Spektrums der Substanz unter Verwendung einer angelernten Komponente maschinellen Lernens zu ermitteln.

Vorteilhafterweise kann mit dieser Vorrichtung der YSI schneller, einfacher, kostengünstiger und genauer ermittelt werden.

Weiter vorteilhafterweise können durch die erfindungsgemäße Vorrichtung Nichtlinearitäten in der Bestimmung des YSI von komplexeren Substanzen wie zum Beispiel Kraftstoffen berücksichtigt und/oder erkannt werden. Solche Substanzen enthalten nämlich in der Regel Hunderte von chemischen Komponenten, die sich gegenseitig beeinflussen und zu Nichtlinearitäten bei der Bestimmung des YSI führen können.

Des Weiteren müssen im Gegensatz zu herkömmlichen Messvorrichtungen Stoffmengenanteile der Einzelkomponenten nicht bestimmt werden bzw. bekannt sein. Die Konzentrationsinformation ist in dem gemessenen Spektrum enthalten und kann somit von der Komponente maschinellen Lernens in die Berechnungen mit einbezogen werden.

Die Anschaffung eines teuren Chromatographen und die zugehörige Methodenentwicklung zur quantitativen Aufklärung der Einzelsubstanzen entfallen damit.

Ein weiterer Vorteil der erfindungsgemäßen Vorrichtung ist, dass im Gegensatz zu herkömmlichen Bestimmungsvorrichtung, die zum Beispiel einen Yale-Brenners umfassen, die erfindungsgemäße Vorrichtung einen einfacheren apparativen Aufbau aufweist. Denn herkömmliche Bestimmungsvorrichtungen sind wesentlich komplexer in ihrem Aufbau und umfassen in der Regel: einen Yale-Brenner, optional einen Laser, Optik für Strahlenführung, -fokussierung und -filterung, einen Photomultiplier als Detektor, ein Oszilloskop zur Signalprüfung und -einstellung, eine Gasversorgung (Stickstoff, Methan, Luft) aus Druckbehältern, eine Probenvorlage als Druckbehälter, eine Pumpe für die Probe, einen "Mass Flow Controller" für die Gasflüsse, einen Datenlogger, sowie einen Computer.

Alle in Bezug auf das Verfahren zur Ermittlung der Rußtendenz einer Substanz gemachten Erläuterungen, beschriebenen Merkmale, Ausführungsformen und Vorteile können, sofern passend, auch auf den Aspekt der Vorrichtung angewendet werden.

Gemäß einer besonderen Ausführungsform umfasst die Ermittlungseinheit einen Desktop-PC und/oder ein Smartphone und/oder einen Tablet-PC. Alternativ kann die Ermittlungseinheit ein Desktop-PC und/oder ein Smartphone und/oder ein Tablet-PC sein.

In dieser Ausführungsform kann die Vorrichtung in bestehende Laborarchitekturen einfach integriert oder implementiert werden.

Gemäß einer besonderen Ausführungsform ist die Vorrichtung dazu ausgelegt, von einem menschlichen Nutzer getragen zu werden. Die Vorrichtung kann ferner Mittel zum Tragen bzw. Transportieren der Vorrichtung durch einen menschlichen Nutzer umfassen. Die Mittel können zum Beispiel eine Halterung für einen Tragegurt und/oder ein Tragegurt und/oder ein oder mehrere Tragegriffe sein.

Vorteilhafterweise kann die Vorrichtung so mobil genutzt werden, um den YSI von Substanzen zu ermitteln.

In einer Ausführungsform sind das Spektrometer und die Ermittlungseinheit kabelgebunden und/oder drahtlos derart miteinander verbunden, dass spektrometrische Messdaten von dem Spektrometer an die Ermittlungseinheit übertragen werden können und/oder die Ermittlungseinheit die spektrometrischen Messdaten von dem Spektrometer abrufen kann.

Die spektrometrischen Messdaten können zum Beispiel die Intensitäten in Abhängigkeit der Wellenzahl oder Wellenlänge der absorbierten und/oder transmittierten elektromagnetischen Strahlung umfassen.

In einer Ausführungsform umfasst die Vorrichtung ferner ein Gehäuse, wobei das Spektrometer und die Ermittlungseinheit in dem Gehäuse angeordnet sind.

In einer Ausführungsform ist an dem Gehäuse ein Bildschirm angeordnet, über den der ermittelte YSI darstellbar ist. Über den Bildschirm kann ferner ein Nutzer mit der Vorrichtung interagieren und zum Beispiel die Messung des Spektrums und/oder die Ermittlung des YSI der Substanz initiieren.

Ein weiterer Aspekt der vorliegenden Erfindung betrifft eine Verwendung der oben beschriebenen Vorrichtung zur Ermittlung der Rußtendenz einer Substanz, insbesondere eines Kraftstoffs und/oder eines Treibstoffs.

Alle in Bezug auf die Vorrichtung beschriebenen Merkmale, Ausführungsformen und Vorteile finden auch Anwendung auf den Aspekt der Verwendung.

Ein weiterer Aspekt der vorliegenden Erfindung betrifft ein Computerprogrammprodukt, umfassend Befehle, die bei der Ausführung des Programms durch einen Computer diesen veranlassen, das oben beschriebene Verfahren zur Ermittlung der Rußtendenz einer Substanz auszuführen.

Alle in Bezug auf das Verfahren beschriebenen Merkmale, Ausführungsformen und Vorteile finden auch Anwendung auf den Aspekt des Computerprogrammprodukts.

Im Folgenden werden spezielle Ausführungsformen der Erfindung anhand der Zeichnungen beschrieben.

**Figur 1** zeigt einen schematischen Verfahrensablauf zur Ermittlung des YSI gemäß einer Ausführungsform der Erfindung.
**Figur 2** zeigt eine Fotografie eines Vorrichtungsaufbaus gemäß einer Ausführungsform der Erfindung.
**Figur 3** zeigt eine Fotografie der Aufgabe einer Probe auf den Kristall eines ATR-FT-IR-Spektrometers.
**Figur 4** zeigt einen Paritätsplot eines Modells zur Vorhersage des YSI auf der Grundlage von Infrarotspektren.
**Figur 5** zeigt den mittleren prozentualen Fehler der Vorhersage von Validierungsdaten für unterschiedliche Anzahlen an Trainingsdatensätzen.
**Figur 6** zeigt eine schematische Darstellung einer Vorrichtung gemäß einer Ausführungsform der Erfindung.
**Figur 7** zeigt ein beispielhaftes System, einschließlich eines Allzweck-Rechengeräts, mit dessen Hilfe die Erfindung umgesetzt werden kann.

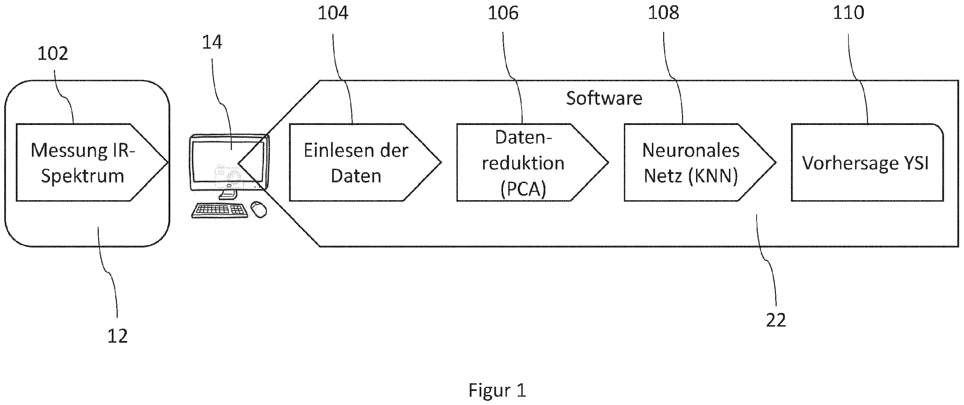

**Figur 1** zeigt einen schematischen Verfahrensablauf zur Ermittlung des YSI gemäß einer Ausführungsform der Erfindung.

Mit einem Abgeschwächte Totalreflexion (ATR)-Fourier-Transform-Infrarot (FT-IR)-Spektrometer 12 wird ein Infrarotspektrum einer Substanz, für die der YSI ermittelt werden soll, aufgenommen (102). Dieses Infrarotspektrum wird, wie in Figur 1 gezeigt, in eine Software 22, die zum Beispiel auf einem Allzweck-Rechner 14 installiert sein kann, der als Ermittlungseinheit fungiert, eingelesen (104).

In oder durch die Software 22 werden die Wellenzahl und die zugehörigen Absorptionswerte aus dem Spektrum eingelesen (104), insgesamt etwa 1800, insbesondere 1762, Datenpaare. Die eingelesenen Daten können zunächst mit etwa 3500 Stützstellen interpoliert werden. Um die Präzision der sich anschließenden Auswertung zu erhöhen und die Rechenzeit zu verringern, kann nun mit den interpolierten Daten eine Hauptkomponentenanalyse durchgeführt werden (106). Dadurch reduziert sich die Anzahl auf insgesamt 100, welche als sogenannte Features in das künstliche neuronale Netzwerk eingespeist werden (108). Das KNN wertet diese im Folgenden aus, indem es vorher antrainierte Muster erkennt und diese einem YSI zuordnet (110).

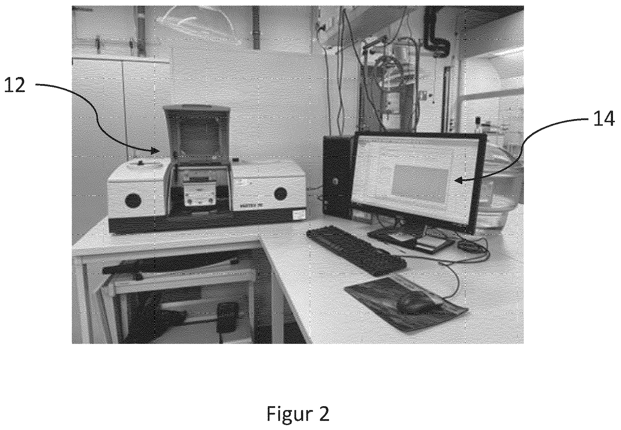

**Figur 2** zeigt eine Fotografie eines Vorrichtungsaufbaus gemäß einer Ausführungsform der Erfindung. Figur 2 zeigt eine Vorrichtung umfassend ein ATR-FT-IR-Spektrometer 12 sowie einen Computer als Ermittlungseinheit 14, welcher die vorgenannte Software 22 einschließlich dem künstlichen neuronalen Netzwerk enthält. Das Spektrometer 12 und die Ermittlungseinheit 14 können als eine Einheit ausgebildet sein. Das Spektrometer 12 und die Ermittlungseinheit 14 können aber auch getrennt voneinander ausgebildet sein.

Das in der Figur 2 gezeigte Infrarotspektrometer 12 ist ein Vertex 70 der Firma Bruker. Die aufgenommenen Spektren werden mit der ebenfalls von der Firma Bruker entwickelten Software OPUS-Operator ausgewertet. Als Computer 14 wird ein windowsbasierter Rechner ohne besondere Leistungsmerkmale verwendet.

Die Software stellt die mit dem Spektrometer gemessenen Absorptionswerte über der Wellenzahl dar. Ebenso führt die Software automatisiert die Hintergrundkorrektur durch, indem vor der eigentlichen Probe eine Messung ohne Probe durchgeführt wird. Die Software wird auch dafür eingesetzt das korrigierte und finale Spektrum als JCAMP-DX-Datei zu speichern.

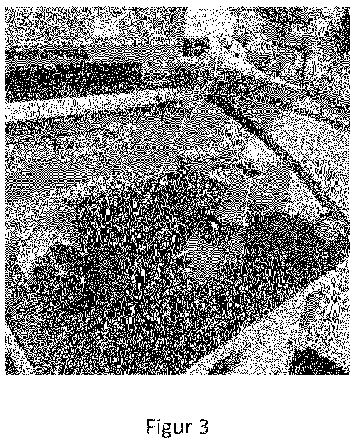

Zunächst wird mit dem Infrarotspektrometer 12 ein Hintergrundabgleich durchgeführt. Dieser Vorgang wird standardmäßig ausgeführt, da sich in der Umgebungsluft infrarotaktive Moleküle wie etwa $CO_2$ befinden, welche ansonsten Fehlersignale verursachen würden. Durch diese Maßnahme kann die Basislinie für die anschließende Messung bestimmt werden. Um die Probe bzw. Substanz, für die der YSI ermittelt werden soll, im Anschluss spektrometrisch messen zu können, wird ein Tropfen von dieser mit einer Pipette auf den ATR-Kristall getropft (siehe **Figur 3,** die eine Fotografie der Aufgabe der Probe bzw. Substanz auf den Kristall des ATR-FT-IR-Spektrometers zeigt).

Hochflüchtige Komponenten mit Siedepunkten kleiner als 40 °C können auch ohne Weiteres vermessen werden. Diese werden, um den Fehler zu minimieren, vor der Messung auf Temperaturen kleiner 0 °C abgekühlt und anschließend aufgegeben. Die spektrometrische Messung wird in der Software gestartet und dauert wenige Sekunden. Das Spektrum wird in einer JCAMP-DX-Datei abgespeichert und kann anschließend mit der Software, mit der das erfindungsgemäße Verfahren, insbesondere die Ermittlung des YSI, umgesetzt werden kann, eingelesen werden. Letztere wertet das gemessene Spektrum aus und gibt den YSI der Probe bzw. Substanz aus.

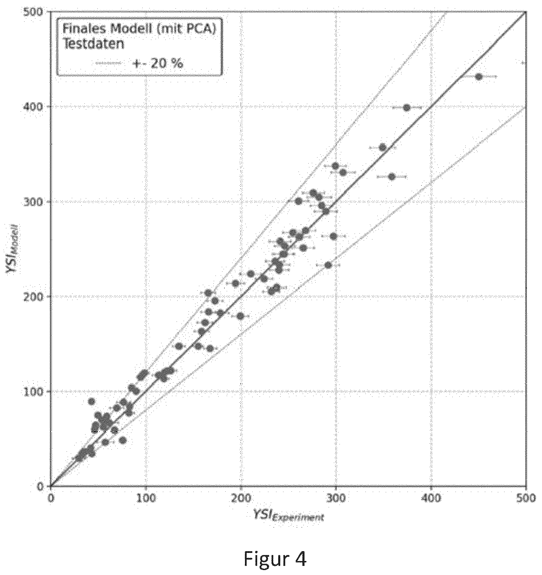

Zum Zeitpunkt der Entstehung dieser Beschreibung wurden 330 Reinsubstanzen sowie Mischungen untersucht, welche im Laufe der Entwicklung zum Training des KNN eingesetzt wurden. Hierfür wurden Aromaten, Paraffine und Cycloalkane im relevanten Bereich von Otto-Kraftstoffen untersucht. In **Figur 4** ist ein Paritätsplot gezeigt, welcher die Vorhersage des YSI mittels des antrainierten Modells bzw. künstlichen neuronales Netzwerkes zeigt. Im Paritätsplot ist der durch das Modell bzw. künstliche neuronale Netzwerk vorhergesagte Wert gegen den experimentellen Referenzwert aufgetragen. Mit dem Modell ist es möglich den YSI für die Testdaten mit einem Fehler von 11 % vorherzusagen. Die sogenannten Testdaten entsprechen Proben, die dem Modell vorher nicht bekannt waren. Das Modell wurde abschließend mit realen Proben aus dem bioliq®-Prozess sowie einer paraffinhaltigen Benzinfraktion aus dem Wachscracking auf Plausibilität geprüft. In der nachfolgenden Tabelle sind die entsprechenden Proben mit ihren petrochemischen Eigenschaften nach DIN EN 228 aufgeführt.

|  | Kraftstoff 1 | Kraftstoff 2 | Kraftstoff 3 |
|---|---|---|---|
| Research Oktanzahl | 101,7 | 74,6 | 79,9 |
| Motor Oktanzahl | 93,7 | 76,8 | 73,9 |
| Dichte in kq m$^{-3}$ | 828,91 | 757,74 | 677,20 |
| Dampfdruck in kPa | 31 | n.v. | n.v. |
| IBP in °C | 41,9 | 45,5 | 34,3 |
| E70 in Vol.-% | 1,1 | 5,5 | 35,1 |
| E100 in Vol.-% | 6,4 | 20,1 | 67,9 |
| E150 in Vol.-% | 85,4 | 66,0 | 96,4 |

(fortgesetzt)

|  | Kraftstoff 1 | Kraftstoff 2 | Kraftstoff 3 |
|---|---|---|---|
| FBP in °C | 171,0 | 162,6 | 168,0 |
| Aromaten in Vol.-% | 76,28 | 1,64 | 0,24 |
| Paraffine in Vol.-% | 16,20 | 18,76 | 95,11 |
| Naphthene in Vol.-% | 5,03 | 79,58 | 4,50 |
| Olefine in Vol.-% | 2,37 | 0,01 | 0,15 |
| Oxygenate in Vol.-% | 0,12 | 0,00 | 0,00 |

Die Abkürzung "n. v." in der oben gezeigten Tabelle steht für "nicht vorhanden".

Alle drei Proben decken gängige Kraft- und Treibstoffe zu weiten Teilen ab, wodurch sie für Validierungszwecke geeignet sind. Die Kraftstoffe wurden dabei so gewählt, dass sie jeweils eine Stoffgruppe (Aromaten, Naphthene und Paraffine) im Überschuss enthalten, um zu überprüfen, wie das Modell mit Randbedingungen zurechtkommt. Für die genannten Proben konnte der genannte Fehlerbereich mit ≤10 % bestätigt werden, womit die Anwendbarkeit auf reale Systeme nachgewiesen werden konnte.

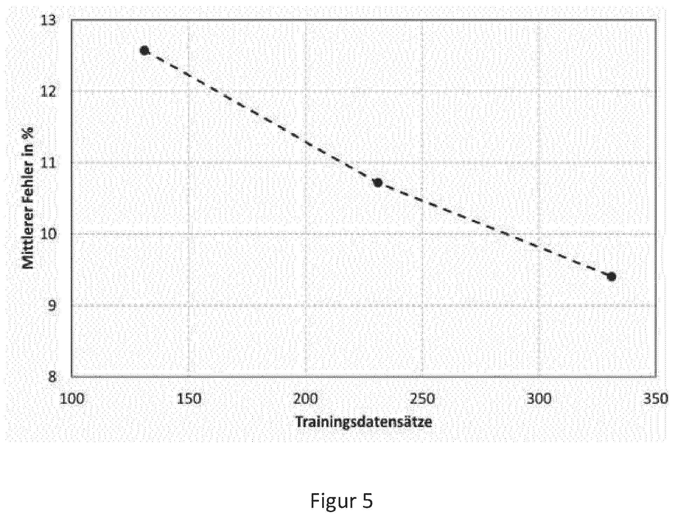

In **Figur 5** ist zu sehen, wie die Anzahl der Datensätze in zwei Schritten um jeweils 100 erhöht wird und der Fehler dadurch im Mittel jeweils um etwa 1,5 % abnimmt. Es ist deshalb anzunehmen, dass zukünftig ein Fehler im Bereich von 6 - 8 % erreichbar sein wird, wenn die Anzahl der Daten verdoppelt wird.

Das hier beschriebene Modell bzw. künstliche neuronale Netzwerk zur Ermittlung bzw. Vorhersage des YSI lässt sich auf weitere in Kraft- und Treibstoffen enthaltene Stoffgruppen wie Olefine und Oxygenate anwenden. Oxygenate wie Ethanol sind häufig verwendete Blending-Komponenten (z. B. E10).

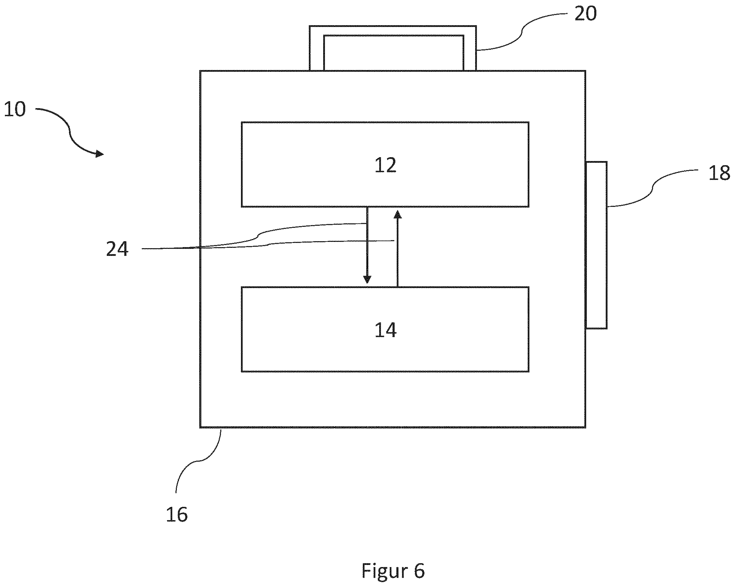

**Figur 6** zeigt eine Ausführungsform der erfindungsgemäßen Vorrichtung 10. Die gezeigte Vorrichtung 10 umfasst erfindungsgemäß ein Spektrometer 12, im Speziellen ein ATR-FT-IR-Spektrometer, und eine Ermittlungseinheit 14, die in der gezeigten Ausführungsform eine Recheneinheit darstellt. Die Recheneinheit 14 weist eine CPU (nicht gezeigt) und einen internen Speicher (nicht gezeigt) auf, in dem die von dem Spektrometer 12 gemessenen Daten (Absorptionswerte und Wellenzahlen) in digitaler Form gespeichert werden können. Das Spektrometer 12 und die Recheneinheit 14 sind über die Datenverbindung 24 miteinander derart verbunden, dass bidirektional Daten zwischen dem Spektrometer 12 und der Recheneinheit 14 ausgetauscht werden können. Ferner kann die Recheneinheit 14 das Spektrometer 12 über die Datenverbindung 24 instruieren, eine Messung zu starten und die spektrometrischen Messdaten an die Recheneinheit zu übermitteln. In dem internen Speicher der Recheneinheit 14 ist die obengenannte Software gespeichert bzw. installiert. Insbesondere ist in dem Speicher das künstliche neuronale Netzwerk abgelegt.

Das Spektrometer 12 kann ebenfalls eine CPU (nicht gezeigt) und einen internen Speicher (nicht gezeigt) aufweisen, in dem die von dem Spektrometer 12 gemessenen Daten einer Probe bzw. Substanz in einer Datei gespeichert werden können. Diese Datei kann dann von dem Spektrometer 12 an die Recheneinheit 14 über die Datenverbindung 24 übergeben oder übermittelt werden oder die Recheneinheit 14 kann die Datei über die Datenverbindung 24 von dem Spektrometer 12 abrufen.

In der in Figur 6 gezeigten Ausführungsform sind das Spektrometer 12 und die Rechen- bzw. Ermittlungseinheit 14 in einem Gehäuse 16 angeordnet. Das Gehäuse 16 umgibt das Spektrometer 12 und die Recheneinheit 14. Das Gehäuse 16 kann bewirken, dass die Vorrichtung 10 eine kompaktere Form hat. Das Gehäuse 16 weist ferner einen oder mehrere Tragegriffe 20 auf, durch den oder die die Vorrichtung 10 von einem menschlichen Nutzer getragen werden kann und mobil eingesetzt werden kann.

Ferner kann die Vorrichtung 10 eine Probenaufnahme (nicht gezeigt) aufweisen, durch die eine Substanz, für die der YSI ermittelt werden soll, dem Spektrometer 12 bereitgestellt werden kann. Die Probenaufnahme kann zum Beispiel eine Öffnung oder Ausnehmung in dem Gehäuse 16 und einen Probenträger oder -halter umfassen, in oder an dem zum Beispiel eine die Substanz enthaltende Küvette angeordnet werden kann.

Außerdem weist die in Figur 6 gezeigte Ausführungsform der Vorrichtung 10 einen Bildschirm 18 auf, über den der durch die Recheneinheit 14 ermittelte YSI der Substanz dargestellt werden kann. Ferner können über den Bildschirm 18 weitere für die Ermittlung des YSI relevante Daten angezeigt werden, wie zum Beispiel Modelldaten des künstlichen neuronalen Netzwerkes. Der Bildschirm 18 ist an dem Gehäuse 16 der Vorrichtung 10 angeordnet.

Die Vorrichtung 10 kann auch einen Ein- und Ausschalter (nicht gezeigt) aufweisen, über den die Stromversorgung der Vorrichtung 10 aktiviert oder deaktiviert werden kann.

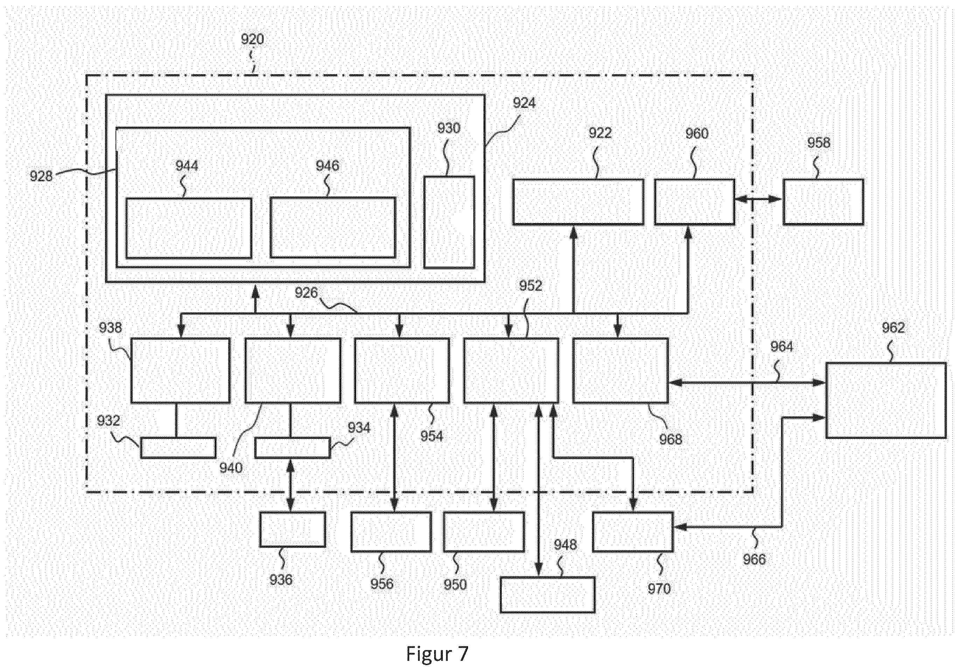

**Figur 7** zeigt ein beispielhaftes System zur Umsetzung der Erfindung, das ein Allzweck-Rechengerät in Form einer herkömmlichen Rechenumgebung 920 (z. B. einen Personalcomputer) enthält, welche als die im Zusammenhang mit den obigen Ausführungsformen beschriebene Ermittlungseinheit 14 verwendet werden kann.

Die herkömmliche Rechenumgebung umfasst eine Verarbeitungseinheit 922, einen Systemspeicher 924 und einen Systembus 926. Der Systembus verbindet verschiedene Systemkomponenten, darunter den Systemspeicher 924, mit der Verarbeitungseinheit 922. Die Verarbeitungseinheit 922 kann arithmetische, logische und/oder Steuerungsoperationen durch Zugriff auf den Systemspeicher 924 durchführen. Der Systemspeicher 924 kann Informationen und/oder Anweisungen zur Verwendung in Kombination mit der Verarbeitungseinheit 922 speichern.

Der Systemspeicher 924 kann einen flüchtigen und einen nichtflüchtigen Speicher umfassen, beispielsweise einen Direktzugriffsspeicher (RAM) 928 und einen Festwertspeicher (ROM) 930. Im ROM 930 kann ein grundlegendes Eingabe-/Ausgabesystem (BIOS) gespeichert sein, das die grundlegenden Routinen enthält, die bei der Übertragung von Informationen zwischen Elementen innerhalb des Personalcomputers 920 helfen, z. B. beim Hochfahren. Der Systembus 926 kann aus verschiedenen Arten von Busstrukturen bestehen, einschließlich eines Speicherbusses oder Speicher-Controllers, eines Peripherie-Busses und eines lokalen Busses, der verschiedene Busarchitekturen verwendet.

Der Personalcomputer 920 kann außerdem ein Festplattenlaufwerk 932 zum Lesen und Beschreiben einer Festplatte (nicht dargestellt) und ein externes Plattenlaufwerk 934 zum Lesen und Beschreiben einer Wechselplatte 936 umfassen. Bei der Wechselplatte kann es sich um eine Magnetplatte für einen Magnetplattentreiber oder um eine optische Platte wie eine CD-ROM für ein optisches Laufwerk handeln. Das Festplattenlaufwerk 932 und das externe Plattenlaufwerk 934 sind über eine Festplattenlaufwerkschnittstelle 938 bzw. eine externe Plattenlaufwerkschnittstelle 940 mit dem Systembus 926 verbunden. Die Laufwerke und die ihnen zugeordneten computerlesbaren Medien dienen der nichtflüchtigen Speicherung von computerlesbaren Anweisungen, Datenstrukturen, Programmmodulen und anderen Daten für den Personalcomputer 920. Die Datenstrukturen können relevante Daten für die Durchführung des erfindungsgemäßen Verfahrens enthalten. Die relevanten Daten können in einer Datenbank organisiert werden, zum Beispiel in einem relationalen Datenbankmanagementsystem oder einem objektorientierten Datenbankmanagementsystem.

Obwohl die hier beschriebene beispielhafte Umgebung eine Festplatte (nicht abgebildet) und eine externe Platte 936 verwendet, sollte dem Fachmann klar sein, dass auch andere Arten von computerlesbaren Medien, die Daten speichern können, auf die ein Computer zugreifen kann, wie z. B. Magnetkassetten, Flash-Speicherkarten, digitale Videodisks, Direktzugriffsspeicher, Festwertspeicher und dergleichen, in der beispielhaften Betriebsumgebung verwendet werden können.

Auf der Festplatte, der externen Festplatte 936, dem ROM 930 oder dem RAM 928 kann eine Reihe von Programmmodulen gespeichert werden, darunter ein Betriebssystem (nicht dargestellt), ein oder mehrere Anwendungsprogramme 944, andere Programmmodule (nicht dargestellt) und Programmdaten 946.

Ein Benutzer kann Befehle und Informationen, wie unten beschrieben, über Eingabegeräte wie die Tastatur 948 und die Maus 950 in den Personalcomputer 920 eingeben. Andere Eingabegeräte (nicht dargestellt) können ein Mikrofon (oder andere Sensoren), einen Joystick, ein Gamepad, einen Scanner oder Ähnliches umfassen. Diese und andere Eingabegeräte können über eine serielle Schnittstelle 952, die mit dem Systembus 926 gekoppelt ist, an die Verarbeitungseinheit 922 angeschlossen werden, oder über andere Schnittstellen, wie eine parallele Schnittstelle 954, einen Spieleanschluss oder einen universellen seriellen Bus (USB), erfasst werden. Außerdem können die Informationen über den Drucker 956 ausgedruckt werden. Der Drucker 956 und andere parallele Eingabe-/Ausgabegeräte können über die parallele Schnittstelle 954 an die Verarbeitungseinheit 922 angeschlossen werden. Ein Monitor 958 oder eine andere Art von Anzeigegerät ist ebenfalls über eine Schnittstelle, wie z. B. einen Videoeingang/-ausgang 960, mit dem Systembus 926 verbunden. Neben dem Monitor kann die Computerumgebung 920 auch andere periphere Ausgabegeräte (nicht abgebildet) umfassen, z. B. Lautsprecher oder andere akustische Ausgänge.

Die Computerumgebung 920 kann mit anderen elektronischen Geräten kommunizieren, z. B. mit einem Computer, einem (drahtgebundenen oder drahtlosen) Telefon, einem persönlichen digitalen Assistenten, einem Fernseher oder ähnlichem. Um zu kommunizieren, kann die Computerumgebung 920 in einer vernetzten Umgebung arbeiten, die Verbindungen zu einem oder mehreren elektronischen Geräten nutzt. Figur 7 zeigt die Computerumgebung, die mit dem entfernten Computer 962 vernetzt ist. Der entfernte Computer 962 kann eine andere Computerumgebung sein, wie z. B. ein Server, ein Router, ein Netzwerk-PC, ein Peer-Gerät oder ein anderer gemeinsamer Netzwerkknoten, und kann viele oder alle der oben beschriebenen Elemente in Bezug auf die Computerumgebung 920 enthalten. Die in Figur 7 dargestellten logischen Verbindungen umfassen ein lokales Netzwerk (LAN) 964 und ein Weitverkehrsnetz (WAN) 966. Solche Netzwerkumgebungen sind in Büros, unternehmensweiten Computernetzwerken, Intranets und dem Internet üblich und können insbesondere verschlüsselt sein.

Bei Verwendung in einer LAN-Netzwerkumgebung kann die Computerumgebung 920 über eine Netzwerk-E/A 968 mit dem LAN 964 verbunden sein. Bei Verwendung in einer WAN-Netzwerkumgebung kann die Computerumgebung 920 ein Modem 970 oder andere Mittel zur Herstellung der Kommunikation über das WAN 966 enthalten. Das Modem 970, das intern oder extern zur Computerumgebung 920 sein kann, ist über die serielle Schnittstelle 952 mit dem Systembus 926 verbunden. In einer vernetzten Umgebung können die in Bezug auf die Computerumgebung 920 dargestellten Programmmodule oder Teile davon in einem entfernten Speichergerät gespeichert werden, das sich auf dem entfernten Computer 962 befindet oder für diesen zugänglich ist. Darüber hinaus können andere Daten, die für das erfindungsgemäße Verfahren (wie oben beschrieben), insbesondere das Ermitteln des YSI, relevant sind, auf dem entfernten Computer 962 gespeichert oder über diesen zugänglich sein. Es wird deutlich, dass die dargestellten Netzwerkverbindungen beispielhaft sind und dass auch andere Mittel zur Herstellung einer Kommunikationsverbindung zwischen den elektronischen Geräten verwendet werden können.

Das oben beschriebene Rechnersystem ist nur ein Beispiel für ein Rechnersystem, das zur Durchführung des erfindungsgemäßen Verfahrens verwendet werden kann.

Zusammenfassend kann mit der oben beschriebenen Erfindung eine Vorrichtung und ein Verfahren bereitgestellt werden, die in bestehende Laborarchitekturen einfach implementiert werden können.

Zudem kann durch die Erfindung der YSI schneller, einfacherer, kostengünstiger und genauer ermittelt werden.

Durch die Erfindung können Nichtlinearitäten in der Bestimmung des YSI von komplexeren Substanzen wie zum Beispiel Kraftstoffen berücksichtigt und erkannt werden. Solche Substanzen enthalten nämlich in der Regel Hunderte von chemischen Komponenten, die sich gegenseitig beeinflussen und zu Nichtlinearitäten bei der Bestimmung des YSI führen können.

Des Weiteren müssen im Gegensatz zu herkömmlichen Bestimmungsmethoden Stoffmengenanteile der Einzelkomponenten nicht bestimmt werden bzw. bekannt sein. Die Konzentrationsinformation ist in dem gemessenen Spektrum enthalten und kann somit von dem künstlichen neuronalen Netzwerk in die Berechnungen mit einbezogen werden.

Die Anschaffung eines teuren Chromatographen und die zugehörige Methodenentwicklung zur quantitativen Aufklärung der Einzelsubstanzen entfallen damit.

Ein weiterer Vorteil der Erfindung ist, dass im Gegensatz zu herkömmlichen Bestimmungsverfahren zum Beispiel mittels eines Yale-Brenners die Erfindung einen einfacheren apparativen Aufbau erlaubt.

Bezugszeichenliste

| | |
|---|---|
| 10 | Vorrichtung |
| 12 | Spektrometer |
| 14 | Ermittlungseinheit |
| 16 | Gehäuse |
| 18 | Bildschirm |
| 20 | Mittel zum Tragen der Vorrichtung |
| 22 | Software |
| 24 | Datenverbindung |
| 920 | Rechenumgebung |
| 922 | Verarbeitungseinheit |
| 924 | Systemspeicher |
| 926 | Systembus |
| 928 | Direktzugriffsspeicher (RAM) |
| 930 | Festwertspeicher (ROM) |
| 932 | Festplattenlaufwerk |
| 934 | externes Plattenlaufwerk |
| 936 | Wechselplatte |
| 938 | Festplattenlaufwerkschnittstelle |
| 940 | externe Plattenlaufwerkschnittstelle |
| 944 | ein oder mehrere Anwendungsprogramme |
| 946 | Programmdaten |
| 948 | Tastatur |
| 950 | Maus |
| 952 | serielle Schnittstelle |
| 954 | parallele Schnittstelle |
| 956 | Drucker |
| 958 | Monitor |
| 960 | Videoeingang/-ausgang |
| 962 | entfernter Computer |
| 964 | lokales Netzwerk (LAN) |
| 966 | Weitverkehrsnetz (WAN) |
| 968 | Netzwerk-E/A |
| 970 | Modem |

**Patentansprüche**

1. Verfahren zur Ermittlung der Rußtendenz einer Substanz, insbesondere eines Kraftstoffs und/oder Treibstoffs, wobei das Verfahren die folgenden Schritte umfasst:

   - Messen (102) eines Spektrums der Substanz mittels eines Spektrometers (12), und
   - Ermitteln (104, 106, 108, 110), mittels einer Ermittlungseinheit (14), des Yield Sooting Index', YSI, der Substanz anhand des gemessenen Spektrums unter Verwendung einer angelernten Komponente maschinellen Lernens.

2. Verfahren nach Anspruch 1, wobei die Komponente maschinellen Lernens ein künstliches neuronales Netzwerk ist.

3. Verfahren nach einem der Ansprüche 1 bis 2, wobei das Verfahren ferner umfasst:
   Anlernen der Komponente maschinellen Lernens, insbesondere mittels:

   gemessener Spektren einer Vielzahl von Referenzsubstanzen und/oder berechneter YSI-Werte der Vielzahl von Referenzsubstanzen, und/oder
   berechneter Spektren der Vielzahl von Referenzsubstanzen und/oder gemessener YSI-Werte der Vielzahl von Referenzsubstanzen.

4. Verfahren nach Anspruch 3, wobei jede Referenzsubstanz der Vielzahl von Referenzsubstanzen bis zu acht chemische Komponenten enthält.

5. Verfahren nach einem der Ansprüche 3 bis 4, wobei das Verfahren ferner umfasst:
   Weiteranlernen der Komponente maschinellen Lernens mittels des gemessenen Spektrums der Substanz und/oder des ermittelten YSI der Substanz.

6. Verfahren nach einem der Ansprüche 1 bis 5, wobei das Spektrum der Substanz ein Absorptionsspektrum, insbesondere ein Infrarotabsorptionsspektrum, und/oder ein Transmissionsspektrum, insbesondere ein Infrarottransmissionsspektrum, ist.

7. Verfahren nach einem der Ansprüche 1 bis 6, wobei das gemessene Spektrum der Substanz bis zu ungefähr 3500 Stützstellen umfasst.

8. Verfahren nach einem der Ansprüche 1 bis 7, wobei das Spektrometer (12) ein Infrarotspektrometer, insbesondere ein Abgeschwächte-Totalreflexion-Fourier-Transform-Infrarotspektrometer, ist.

9. Verfahren nach einem der Ansprüche 1 bis 8, wobei das Verfahren ferner umfasst:

   Durchführen einer Datenreduktion (106) des gemessenen Spektrums der Substanz, insbesondere einer Hauptkomponentenanalyse des gemessenen Spektrums der Substanz.

10. Vorrichtung (10) zur Ermittlung der Rußtendenz einer Substanz, insbesondere eines Kraftstoffs und/oder Treibstoffs, umfassend:

    - ein Spektrometer (12) zur Messung eines Spektrums der Substanz, und
    - eine Ermittlungseinheit (14), die dazu ausgelegt ist, den Yield Sooting Index, YSI, der Substanz anhand des gemessenen Spektrums der Substanz unter Verwendung einer angelernten Komponente maschinellen Lernens zu ermitteln.

11. Verwendung der Vorrichtung (10) nach Anspruch 10 zur Ermittlung der Rußtendenz einer Substanz, insbesondere eines Kraftstoffs und/oder Treibstoffs.

12. Computerprogrammprodukt, umfassend Befehle, die bei der Ausführung des Programms durch einen Computer diesen veranlassen, das Verfahren nach einem der Ansprüche 1 bis 9 auszuführen.

Figur 1

Figur 2

Figur 3

Figur 4

Figur 5

Figur 6

Figur 7

EP 4 296 651 A1

Europäisches Patentamt
European Patent Office
Office européen des brevets

## EUROPÄISCHER RECHERCHENBERICHT

Nummer der Anmeldung

EP 23 18 0659

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | ABDUL JAMEEL ABDUL GANI: "Predicting Sooting Propensity of Oxygenated Fuels Using Artificial Neural Networks", PROCESSES, Bd. 9, Nr. 6, 19. Juni 2021 (2021-06-19), Seite 1070, XP093098452, DOI: 10.3390/pr9061070 | 1-5,7, 9-12 | INV. G01N21/35 G06N3/09 ADD. G01N21/552 |
| Y | * Zusammenfassung * <br> * Seite 2, letzter Absatz * <br> * Seite 13, Absatz 2 * <br> ----- | 6,8 | |
| X | ABDUL JAMEEL ABDUL GANI: "Identification and Quantification of Hydrocarbon Functional Groups in Gasoline Using 1H-NMR Spectroscopy for Property Prediction", MOLECULES, Bd. 26, Nr. 22, 19. November 2021 (2021-11-19), Seite 6989, XP093098594, DOI: 10.3390/molecules26226989 Gefunden im Internet: URL:https://www.ncbi.nlm.nih.gov/pmc/artic les/PMC8622857/pdf/molecules-26-06989.pdf> | 1-5,7, 9-12 | |
| Y | * Zusammenfassung * <br> * Seite 7, Absatz 2 * <br> ----- <br> -/-- | 6,8 | RECHERCHIERTE SACHGEBIETE (IPC) <br><br> G01N |

Der vorliegende Recherchenbericht wurde für alle Patentansprüche erstellt

| Recherchenort | Abschlußdatum der Recherche | Prüfer |
|---|---|---|
| München | 8. November 2023 | D'Alessandro, Davide |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

Europäisches
Patentamt

European
Patent Office

Office européen
des brevets

## EUROPÄISCHER RECHERCHENBERICHT

Nummer der Anmeldung

EP 23 18 0659

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WANG YU ET AL: "On estimating physical and chemical properties of hydrocarbon fuels using mid-infrared FTIR spectra and regularized linear models", FUEL, IPC SIENCE AND TECHNOLOGY PRESS , GUILDFORD, GB, Bd. 255, 4. Juli 2019 (2019-07-04), XP085758170, ISSN: 0016-2361, DOI: 10.1016/J.FUEL.2019.115715 [gefunden am 2019-07-04] * Zusammenfassung * * Seite 3, rechte Spalte * ----- | 6,8 | |
| Y | ENDERS ABIGAIL A. ET AL: "Functional Group Identification for FTIR Spectra Using Image-Based Machine Learning Models", ANALYTICAL CHEMISTRY, Bd. 93, Nr. 28, 30. Juni 2021 (2021-06-30) , Seiten 9711-9718, XP093098655, US ISSN: 0003-2700, DOI: 10.1021/acs.analchem.1c00867 * Zusammenfassung * * Tabelle 1 * ----- | 6,8 | RECHERCHIERTE SACHGEBIETE (IPC) |
| Y | TANG B ET AL: "Chemical characterization of oil-based asphalt release agents and their emissions", FUEL, IPC SIENCE AND TECHNOLOGY PRESS , GUILDFORD, GB, Bd. 85, Nr. 9, 1. Juni 2006 (2006-06-01), Seiten 1232-1241, XP028064516, ISSN: 0016-2361, DOI: 10.1016/J.FUEL.2005.11.002 [gefunden am 2006-06-01] * Zusammenfassung * * Seite 1235 * ----- | 6,8 | |

Der vorliegende Recherchenbericht wurde für alle Patentansprüche erstellt

| Recherchenort | Abschlußdatum der Recherche | Prüfer |
|---|---|---|
| München | 8. November 2023 | D'Alessandro, Davide |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
......................................................................
& : Mitglied der gleichen Patentfamilie, übereinstimmendes Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **MCENALLY, CHARLES S. ; DAS, DHRUBAJYOTI D. ; PFEFFERLE, LISA D.** Yield Sooting Index Database. *Sooting Tendencies of a Wide Range of Fuel Compounds on a Unified Scale,* 2007, vol. 2, https://doi.org/10.7910/DVN/7HGFT
- **MCENALLY, CHARLES S. ; PFEFFERLE, LISA D.** Sooting tendencies of oxygenated hydrocarbons in laboratory-scale flames. *Environmental Science & Technology,* 2011, vol. 45 (6), ISSN 0013-936X, 2498-2503
- **DAS, DHRUBAJYOTI D. ; MCENALLY, CHARLES S. ; PFEFFERLE, LISA D.** Sooting tendencies of unsaturated esters in nonpremixed flames. *Combustion and Flame,* 2015, vol. 162 (4), ISSN 0010-2180, 1489-1497
- **MCENALLY, CHARLES S. ; PFEFFERLE, LISA D.** Improved sooting tendency measurements for aromatic hydrocarbons and their implications for naphthalene formation pathways. *Combustion and Flame,* 2007, vol. 148 (4), ISSN 0010-2180, 210-222
- **MCENALLY, CHARLES S. ; PFEFFERLE, LISA D.** Sooting tendencies of nonvolatile aromatic hydrocarbons. *Proceedings of the Combustion Institute,* 2009, vol. 32 (1), ISSN 1540-7489, 673-679
- **DAS, DHRUBAJYOTI D.** Measuring and predicting sooting tendencies of oxygenates, alkanes, alkenes, cycloalkanes, and aromatics on a unified scale. *Combustion and Flame,* 2018, vol. 190, ISSN 0010-2180, 349-364